# EUROPÄISCHE PATENTANMELDUNG

(11) **EP 0 654 535 A1**
(43) Veröffentlichungstag der Anmeldung: **24.05.1995**
(21) Anmeldenummer: 94118134.9
(22) Anmeldetag: 17.11.1994
(51) Int. Cl.: C12Q 1/06

(54) **Keimzahlbestimmungsanordnung für den klinischen Bereich**

(30) Priorität: 18.11.1993 DE 4339378
(71) Anmelder: SIEMENS AKTIENGESELLSCHAFT, D-80333 München (DE)
(72) Erfinder: Sander, Johannes Prof. Dr., D-30952 Ronnenberg-Benthe (DE); Slemeyer, Andreas Prof. Dr., D-35041 Marburg (DE); Ricklefs, Ubbo Prof. Dr., D-35753 Greifenstein (DE); Horn, Horst, D-31832 Springe (DE)

(57) **Zusammenfassung**

Die vorliegende Erfindung betrifft eine Keimzahlbestimmungsanordnung für den klinischen Bereich, insbesondere für Operationssäle (1), zur Überwachung der Luft eines Raumes (1) auf Keime, mit einem Meßvolumen (15), dem auf Keime zu untersuchende Luft aus dem Raum (1) zuführbar ist, einer ortsbeweglichen Sensoranordnung (2), die die Anzahl von in der Luft vorhandenen Partikeln ermittelt, und einer Auswerteeinheit (6), die aus der ermittelten Anzahl von Partikeln die Keimzahl bestimmt.

## Beschreibung

Die vorliegende Erfindung betrifft eine Keimzahlbestimmungsanordnung für den klinischen Bereich, insbesondere für Operationssäle, zur Überwachung der Luft eines Raumes auf Keime, mit einem Meßvolumen, dem auf Keime zu untersuchende Luft aus dem Raum zuführbar ist.

Keime sind, wie allgemein bekannt ist, Verursacher von Infektionen. Insbesondere in Operationssälen, in denen offene Wunden entstehen, ist daher eine zuverlässige, insbesondere laufende, Überwachung der Keimzahl der Raumluft angebracht. Keime in der Raumluft, insbesondere nahe der Operationswunde, sind jedoch nur sehr schwer nachweisbar.

Die Aufgabe der vorliegenden Erfindung besteht darin, erstmals eine einfache und relativ kostengünstige, dennoch im unmittelbaren Operationsbereich zuverlässig und genau arbeitende Keimzahlbestimmungsanordnung zu schaffen. Dabei soll dem Operateur die Möglichkeit gegeben werden, bei Erreichen von kritischen Werten auf das Keimaufkommen einzuwirken bzw. Gegenmaßnahmen zu ergreifen.

Die Aufgabe wird dadurch gelöst, daß die Keimzahlbestimmungsanordnung eine ortsbewegliche Sensoranordnung, die die Anzahl von in der Luft vorhandenen Partikeln ermittelt, und eine Recheneinheit, die aus der ermittelten Anzahl von Partikeln die Keimzahl bestimmt, aufweist.

Überraschenderweise hat sich nämlich herausgestellt, daß Keime nicht selbst in der Luft transportiert werden, sondern fast ausschließlich an Partikel gebunden sind. Es besteht eine eindeutige Korrellation zwischen der Anzahl der in der Raumluft vorhandenen Partikel und der Keimzahl. Als Träger von Keimen kommen dabei hauptsächlich Partikel mit einer Größe zwischen 2 und 20 um in Frage, wobei im Mittel jedes tausendste Partikel tatsächlich einen Keim trägt.

Zur Erhöhung der Betriebssicherheit ist zur Verstopfungserkennung der Sensoranordnung vorzugsweise eine Unterdruckmessung durchführbar.

Wenn die Sensoranordnung innerhalb und die Auswerteeinheit außerhalb des auf Keime zu untersuchenden Raumes angeordnet ist, ist die Einwirkung der Keimzahlbestimmungsanordnung auf den Operationsbereich so gering wie möglich.

Weitere Vorteile und Einzelheiten ergeben sich u.a. aus der nachfolgenden Beschreibung eines besonders kostengünstigen Ausführungsbeispiels, anhand der Zeichnungen und in Verbindung mit den weiteren Unteransprüchen. Dabei zeigen:
- FIG 1: eine Keimzahlbestimmungsanordnung in einem Operationssaal,
- FIG 2 und 3: je eine Streulichtsensoranordnung und
- FIG 4: die Detektoranordnung bei der Streulichtsensoranordnung gemäß FIG 3.

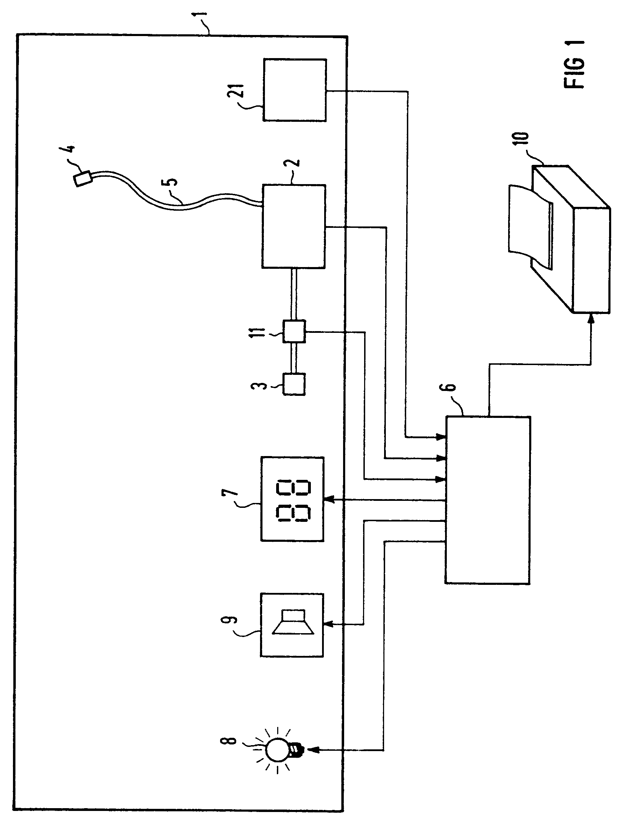

Gemäß FIG 1 ist innerhalb eines Operationssaals 1 eine Sensoranordnung 2 zur Detektierung von Partikeln in der Luft des Operationssaals 1 angeordnet. Die Luft wird dabei über die Pumpe 3 durch die vorzugsweise einfach manuell bewegliche Düse 4 und den Schlauch 5 angesaugt. Die von der Sensoranordnung 2 ermittelte Anzahl von Partikeln in der Raumluft des Operationssaals 1 wird an die Auswerteeinheit 6 (hier einen PC) gemeldet und dort ausgewertet. Insbesondere bestimmt die Auswerteeinheit 6, aufgrund der ermittelten Korrelation zwischen Anzahl von Partikeln und Keimzahl, die Keimzahl in der Raumluft des Operationssaals 1.

Die so bestimmte Keimzahl wird auf der Anzeigeeinheit 7, z.B. einem Leuchtdiodendisplay, dargestellt. Die bestimmte Keimzahl wird auch laufend mit einem vom Operateur vorgebbaren Grenzwert verglichen. Wenn die bestimmte Keimzahl den Grenzwert überschreitet, wird zusätzlich zur Anzeige über die Warnlampe 8 und/oder die Warnhupe 9 ein optisches bzw. akustisches Warnsignal ausgegeben. Ferner ist über die Warnlampe 8 und die Warnhupe 9 auch eine Tendenzanzeige möglich. Desgleichen über einen Drucker.

Die Keimzahl wird immer wieder bestimmt, z.B. jede Minute. Die im Verlauf einer Operation bestimmten Keimzahlen sind daher über die Ausgabeeinheit 10, hier einen Drucker, ausgebbar. Bei jedem Ausdruck werden Datum und Uhrzeit mit ausgedruckt. Dadurch ist der Keimzahlverlauf protokolliert und dokumentiert.

Zwischen der Sensoranordnung 2 und der Pumpe 3 ist eine Unterdruckmeßeinrichtung 11 o.ä. angeordnet, deren Ausgangssignal ebenfalls der Auswerteeinheit 6 zugeführt wird. Dadurch ist erkennbar, wenn in der Düse 4, dem Schlauch 5 oder der Sensoranordnung 2 eine Verstopfung des Luftkanals auftritt. Die Sensoranordnung 2 kann daher laufend auf ihre Funktionsfähigkeit überprüft werden.

In der Nähe der Unterdruckmeßeinrichtung 11 ist auch ein - der Übersichtlichkeit halber nicht dargestellter - heizbarer Halbleiter-Gassensor, z. B. auf SnO₂ - oder ZnO-Basis, angeordnet. Mit diesem Sensor sind sowohl oxidierbare als auch nicht oxidierbare Gase quantitativ bestimmbar. Insbesondere ist mittels des Sensors die Konzentration von Narkosegasen (z. B. Lachgas) meßbar. Diese Konzentration wird ebenfalls auf der Anzeigeeinheit 7 dargestellt. Anstelle des Halbleitersensors kann auch ein elektrochemischer Sensor verwendet werden.

Wie aus FIG 1 ersichtlich ist, sind z.B. sowohl die Sensoranordnung 2 mit ihren Zubehörteilen 3,4,5 und 11 sowie die Anzeigeeinrichtungen 7 bis 9 im Operationssaal 1 angeordnet, während vorteilhaft die Auswerteeinheit 6 und der Drucker 10 außerhalb des Operationssaales 1 angeordnet sind. Dadurch, daß die Auswerteeinheit 6 und der Drucker 10 außerhalb des Operationssaales 1 angeordnet sind, wird die Einwirkung der Keimzahlbestimmungsanordnung, die aus den Elementen 2 bis 11 besteht, auf die Raumluft des Operationssaales 1 minimiert.

Alle innerhalb des Operationssaales 1 angeordneten Elemente der Keimzahlbestimmungsanordnung sind vorteilhaft sterilisierbar ausgebildet. Dadurch können Keime nicht von der Sensoranordnung 2 und ihren Zubehörteilen ausgehen.

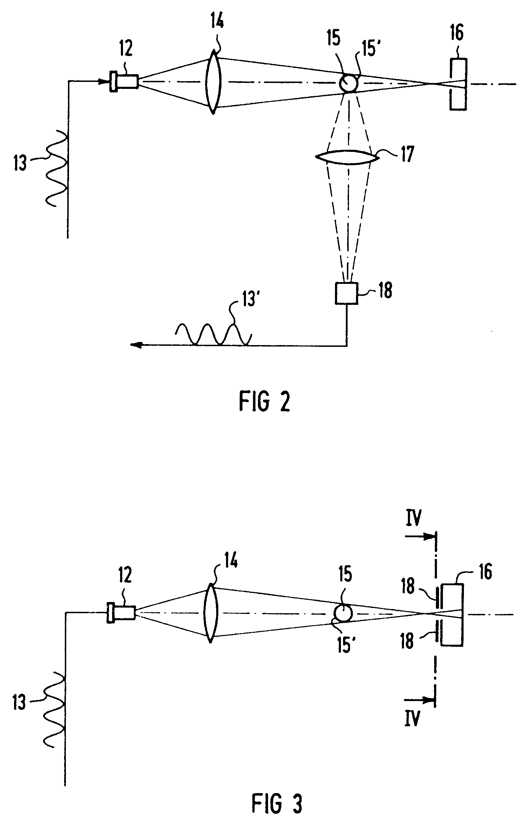

Wie aus FIG 2 ersichtlich ist, ist die Sensoranordnung 2 im vorliegenden Fall als besonders kostengünstige Streulichtsensoranordnung ausgebildet. Hierzu emittiert die Laserdiode 12 Licht. Dieses Licht ist moduliert, wie durch den Wellenzug 13 angedeutet ist. Das modulierte Licht wird durch die Linse 14, die als Kollimieroptik zwischen Laserdiode 12 und Meßvolumen 15 angeordnet ist, kollimiert. Das Meßvolumen 15 wird durch ein durchsichtiges Rohr 15' umgrenzt, durch das von der Laserdiode 12 aus gesehen quer zur optischen Achse die zu untersuchende Luft strömt. Das Rohr 15' kann aber auch entfallen.

Der Großteil des von der Laserdiode 12 emittierten Lichts durchdringt das Rohr 15' ungestreut. Dieser Teil wird durch die Lichtfalle 16 abgeschattet. Vom gestreuten Licht wird ein Teil durch die Linse 17, die als Abbildungsoptik zwischen Rohr 15 und Detektor 18 (hier einer Photodiode) angeordnet ist, auf den Detektor 18 abgebildet. Das vom Detektor 18 detektierte Signal wird der Auswerteeinheit 6 zugeführt. Dieses Signal ist selbstverständlich gemäß dem von der Laserdiode 12 emittierten Licht moduliert, wie durch den Wellenzug 13' angedeutet ist.

Durch die Modulation des von der Laserdiode 12 emittierten Lichts wird das Signal-Rausch-Verhältnis deutlich erhöht. Es ist also mit dem modulierten Licht erheblich leichter, die Anzahl von Partikeln bzw. den Luftdruck im Meßvolumen 15 zu bestimmen, als mit unmoduliertem Licht.

Im Vergleich zu FIG 2 besticht die Streulichtsensoranordnung gemaß FIG 3 durch ihre Einfachheit und Robustheit. Gemäß FIG 3 sind die Detektoren 18 nunmehr nämlich im wesentlichen hinter der Meßzone 15 auf der Lichtfalle 16, allerdings außerhalb des Lichtkegels des ungestreuten Lichts, angeordnet. Zwischen den Detektoren 18 und dem Meßvolumen 15 ist dabei keinerlei abbildende Optik angeordnet.

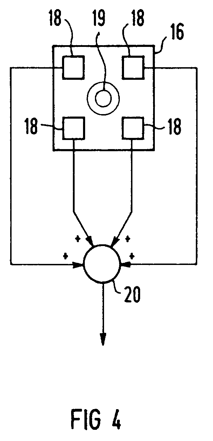

Wie aus FIG 4 ersichtlich ist, sind vier Detektoren 18 ringförmig um den Lichtkegel 19 des ungestreuten Lichts herum angeordnet Die Ausgangssignale dieser Detektoren 18 werden im Additionsglied 20 additiv miteinander verknüpft.

Mit der Anordnung gemäß den FIG 3 und 4 ist eine sehr einfache, kostengünstige Möglichkeit zur Realisierung einer Streulichtsensoranordnung bei dennoch befriedigendem Signal-Rausch-Verhältnis gegeben.

Wie obenstehend erwähnt, wird durch die Sensoranordnung 2 die Anzahl der im Meßvolumen 15 vorhandenen Partikel gemessen und aus diesem Meßwert durch die Auswerteeinheit 6 die Zahl der im Meßvolumen 15 vorhandenen Keime bestimmt. Da sich der Nullpunkt und die Empfindlichkeit der Sensoranordnung 2 im Laufe der Zeit ändern können, wird von Zeit zu Zeit eine Kalibrierung durchgeführt. Diese erfolgt vorteilhaft durch Vergleich mit den Meßwerten eines zertifizierten, z.B. in einem Labor ortsfesten aufgestellten, kalibrierten Gerät, das als Eichnormal dient.

Bei der beschriebenen kostengünstigen Sensoranordnung wird - bei partikelfreier Luft - eine Vergleichsmessung zwischen der Sensoranordnung 2 und dem Luftdruckmesser 21 durchgeführt. Dabei werden der Sensoranordnung 2 und dem Luftdruckmesser 21 eine Reihe von Atmosphären unterschiedlichen Drucks vorgegeben. Die Meßreihen der Sensoranordnung 2 und des Luftdruckmessers 21 werden miteinander verglichen. Zur Erhöhung der Meßgenauigkeit bzw. des Signal-Rausch-Verhältnisses erfolgt auch die Kalibrierung der Sensoranordnung 2 stets mit moduliertem Licht. Aufgrund eines Vergleichs der Meßreihen ist eine Bestimmung der Meßempfindlichkeit und der Nullpunktverschiebung der Sensoranordnung 2 möglich. Anstelle eines direkten Vergleichs der Meßwerte der Sensoranordnung 2 mit den tatsächlichen Luftdrücken können die Meßwerte selbstverständlich auch mit Meßwerten verglichen werden, die früher von der Sensoranordnung 2 gemessen wurden.

Die Kalibrierung der Sensoranordnung 2 beruht dabei auf der Erkenntnis, daß - wenn auch nur in geringem Umfang - Licht auch an Luftmolekülen gestreut wird. Wenn also aufgrund eines höheren Druckes mehr Luftmoleküle im Meßvolumen 15 vorhanden sind, so wird auch mehr Licht gestreut. Messungen ergeben eine Stromänderung von 5 % bei einer Druckänderung von 500 hPa. Damit sind entweder der Luftdruck oder - wie im vorliegenden Fall - bei bekanntem Luftdruck Nullpunktstrom und Empfindlichkeit der Streulichtsensoranordnung 2 auf ca. 1 ^{‰} genau bestimmbar.

Besonders genau und damit insbesondere für Operationssäle der Neurochirurgie und der Knochenchirurgie geeignet, ist die Verwendung eines direkten Partikelzählgeräts zur Keimzahlbestimmung. Bei derartigen Geräten wird der Luftstrom des Meßgerätes derartig konditioniert, daß einzelne Partikel gemessen werden können. So ist eine exakte Kontrolle der zur Reduzierung der Keimzahl im Operationssaal am Operationsort getroffenen Maßnahmen möglich. Falls es sich ergeben sollte, daß die Keimzahl den Meßbereich dieses sehr genauen Gerätes übersteigt, ertönt ein Alarmsignal. Das Alarmsignal zeigt gleichzeitig an, daß Maßnahmen zur Herabsetzung der aktuellen Keimzahl getroffen werden müssen, z.B. durch Spülung des Operationsortes mit keimfreier "künstlicher" Luft.

Besonders vorteilhaft ist bei dem Einzel-Zähl-Sensor, daß Partikel unterschiedlicher Größe in unterschiedlichen Kanälen gemessen werden können, da so auch eine Abschätzung der Größe der im Raum enthaltenen Keime möglich ist.

Um eine Suche nach Keimquellen zu erleichtern, ist das Gerät vorteilhaft mit einer akustischen Anzeige der Keimzahl, ähnlich einem Geigerzähler ausgerüstet. So ist eine einfache Mittlung der Quellen und Senken in der Keimverteilung möglich.

## Patentansprüche

1. Keimzahlbestimmungsanordnung für den klinischen Bereich, insbesondere für Operationssäle (1), zur Überwachung der Luft eines Raumes (1) auf Keime, mit
- einem Meßvolumen (15), dem auf Keime zu untersuchende Luft aus dem Raum (1) zuführbar ist,
- einer ortsbeweglichen Sensoranordnung (2), die die Anzahl von in der Luft vorhandenen Partikeln ermittelt, und
- einer Auswerteeinheit (6), die aus der ermittelten Anzahl von Partikeln die Keimzahl bestimmt.

2. Keimzahlbestimmungsanordnung nach Anspruch 1,
**dadurch gekennzeichnet**,
daß die Sensoranordnung (2) als Streulichtsensoranordnung (2) ausgebildet ist.

3. Keimzahlbestimmungsanordnung nach Anspruch 1,
**dadurch gekennzeichnet**,
daß die Sensoranordnung (2) als direkte Partikel-Zählanordnung ausgebildet ist.

4. Keimzahlbestimmungsanordnung nach Anspruch 1,2 oder 3,
**dadurch gekennzeichnet**,
daß sie zur Messung von unterschiedlichen Partikelgrößen mehrkanalig ausgebildet ist, etwa zur Messung von Partikelgrößen 0,3 µm, 0,5 µm, 5,0 und 10,0 µm.

5. Keimzahlbestimmungsanordnung nach Anspruch 3 oder 4,
**dadurch gekennzeichnet**,
daß die Luft dem Meßvolumen (15) über einen Schlauch (5) zuführbar ist.

6. Keimzahlbestimmungsanordnung nach Anspruch 3,4 oder 5,
**dadurch gekennzeichnet**,
daß zur Verstopfungserkennung der Sensoranordnung (2) eine Unterdruckmessung durchführbar ist.

7. Keimzahlbestimmungsanordnung nach einem der obigen Ansprüche, **dadurch gekennzeichnet**,
daß sie eine Anzeigeeinheit (7) zum Anzeigen der bestimmten Keimzahl aufweist.

8. Keimzahlbestimmungsanordnung nach einem der obigen Ansprüche, **dadurch gekennzeichnet**,
daß sie einen optisch und/oder akustisch wirkenden Signalgeber (8,9) zur Ausgabe eines Warnsignals aufweist.

9. Keimzahlbestimmungsanordnung nach einem der obigen Ansprüche, **dadurch gekennzeichnet**, daß sie eine Ausgabeeinheit (10), z.B. einen Drucker (10), etwa zum Protokollieren und/oder Dokumentieren der bestimmten Keimzahl während der Operationsdauer aufweist.

10. Keimzahlbestimmungsanordnung nach einem der obigen Ansprüche, **dadurch gekennzeichnet**,
daß zumindest die Sensoranordnung (2) innerhalb und zumindest die Auswerteeinheit (6) außerhalb des auf Keime zu untersuchenden Raumes (1) angeordnet sind.

11. Keimzahlbestimmungsanordnung nach Anspruch 10,
**dadurch gekennzeichnet**,
daß die innerhalb des auf Keime zu untersuchenden Raumes angeordneten Elemente (z.B. 2,3,11) der Keimzahlbestimmungsanordnung sterilisierbar sind.

12. Keimzahlbestimmungsanordnung nach einem der obigen Ansprüche, **dadurch gekennzeichnet,**
daß sie einen Gassensor, insbesondere einen Halbleiter-Gassensor, zur Bestimmung von Narkosegasen aufweist.

13. Keimzahlbestimmungsanordnung nach einem der obigen Ansprüche, **dadurch gekennzeichnet,**
daß sie in geeichter Form verwendet wird, insbesondere durch eine periodisch wiederholte Vergleichsmessung mit einem stationären, zertifizierten Partikelzählgerät.

14. Keimzahlbestimmung für den klinischen Bereich, vorzugsweise von Operationssälen, insbesondere nach einem oder mehreren der vorhergehenden Ansprüche,
**dadurch gekennzeichnet,**
daß die Keimzahl anhand ihrer Korrelation zu den in der Raumluft enthaltenen Partikeln bestimmt wird.
